# EUROPEAN PATENT APPLICATION

(11) **EP 4 525 021 A1**
(43) Date of publication of application: **19.03.2025**
(21) Application number: 23842615.9
(22) Date of filing: 06.03.2023
(51) Int. Cl.: H01J 65/00, A61L 2/10, H01J 61/56

(54) **ULTRAVIOLET-LIGHT-RADIATING DEVICE, AND METHOD FOR CONTROLLING ULTRAVIOLET-LIGHT-RADIATING DEVICE**

(30) Priority: 22.07.2022 JP 2022117011
(71) Applicant: Jamco Corporation, Mitaka-shi, Tokyo 181-8571 (JP); Ushio Denki Kabushiki Kaisha, Tokyo 100-8150 (JP)
(72) Inventor: OSHITA, Toshihiko, Tachikawa-shi, Tokyo 190-0011 (JP); ASAKAWA, Seiji, Tachikawa-shi, Tokyo 190-0011 (JP); IMAMURA, Atsushi, Tokyo 100-8150 (JP); YAGYU, Hideaki, Tokyo 100-8150 (JP)
(74) Representative: Novaimo
(86) International application number: PCT/JP2023/008299
(87) International publication number: WO 2024/018678

(57) **Abstract**

The present invention provides an ultraviolet-light-radiating device that may irradiate ultraviolet light stably even in a low air pressure environment. To achieve this object, one representative ultraviolet-light-radiating device according to the present invention includes a radiating unit configured to irradiate ultraviolet light, a first detection unit configured to detect a dielectric strength of air in an atmosphere in which the ultraviolet-light-radiating device is disposed; and a control unit configured to control the radiating unit based on the dielectric strength of air detected by the first detection unit.

## Description

### [Technical Field]

The present invention relates to ultraviolet-light-radiating devices, and methods for controlling ultraviolet-light-radiating devices.

### [Background Art]

With the global spread of COVID-19 infection, there are increasing demands for a sterilization treatment utilizing ultraviolet lamps, and demands for ultraviolet lamps are increasing not only in medical fields but also in various living environments.

There are ultraviolet lamps with emission spectrums of various wavelengths, but some may have adverse effects when irradiated on a human body. Therefore, in medical fields, ultraviolet light with a wavelength of 207 nm or more and 220 nm or less is used to perform sterilization treatment while avoiding risks of harming the human body.

Patent Literature 1 discloses a compact ultraviolet-light-radiating device in which a degree of adverse effects on the human body is suppressed.

Patent Literature 1 discloses an ultraviolet-light-radiating device including "a lamp house on at least one surface of which is formed a light extraction surface, an excimer lamp accommodated in the lamp house that emits ultraviolet light whose main emission wavelength belongs to a first wavelength band of 190 nm or more and 225 nm or less, a first electrode and a second electrode that are disposed in contact with an outer surface of a luminous tube of the excimer lamp, an optical filter that is arranged on the light extraction surface and that substantially transmits ultraviolet light in the first wavelength band and that substantially reflects ultraviolet light of a wavelength of 240 nm or more and 300 nm or less, and a reflecting surface that is a surface located outside the luminous tube and inclined with respect to the light extraction surface as seen in a tube axis direction of the luminous tube of the excimer lamp, the reflecting surface exhibiting reflectivity with respect to ultraviolet light in the first wavelength band".

### [Citation List]

### [Patent Document]

[Patent Document 1] WO2021/070780

### [Disclosure of the Invention]

### [Problems to be Solved by the Invention]

When the needs for sterilization treatment by ultraviolet lamps have increased, and it has become possible to utilize the ultraviolet-light-radiating device under various environments due to the downsizing of the radiating device, utilization of the ultraviolet-light-radiating device may be attempted even under environments that have not yet been assumed. For example, even in aircrafts of international airlines that are used by many passengers, the need for sterilization treatments using ultraviolet-light-radiating devices has increased from the viewpoint of preventing the spread of infection of COVID-19.

However, air pressure within a cabin of aircrafts during flight may become lower than on the ground, and the influence of such low air pressure environment on the ultraviolet-light-radiating device is not investigated in Patent Literature 1.

Therefore, the present invention aims at providing an ultraviolet-light-radiating device that may irradiate ultraviolet light stably even in a low air pressure environment.

### [Means of Solving the Problems]

In order to solve the problems described above, one representative ultraviolet-light-radiating device according to the present invention includes a radiating unit configured to irradiate ultraviolet light, a first detection unit configured to detect a dielectric strength of air in an atmosphere in which the ultraviolet-light-radiating device is disposed, and a control unit configured to control the radiating unit based on the dielectric strength of air detected by the first detection unit.

### [Effects of the Invention]

According to the present invention, it becomes possible to provide an ultraviolet-light-radiating device that may irradiate ultraviolet light stably even in a low air pressure environment.

Problems, configurations and effects other than those described above will become apparent by the description of embodiments for carrying out the invention.

### [Brief Description of the Drawings]

FIG. 1 is a perspective view schematically illustrating an external appearance of an ultraviolet-light-radiating device.
FIG. 2 is a perspective view having exploded a main body casing portion and a lid portion of a lamp house from the ultraviolet-light-radiating device of FIG. 1.
FIG. 3 is a perspective view schematically illustrating a structure of an electrode block and excimer lamps equipped in the ultraviolet-light-radiating device.
FIG. 4 is a perspective view from a viewpoint different from FIG. 3.
FIG. 5 is a perspective view having omitted the excimer lamps from FIG. 4.
FIG. 6 is a view schematically illustrating a positional relationship between the excimer lamps and the electrode block.
FIG. 7 is a view illustrating one example of a configuration of an ultraviolet-light-radiating device according to a first embodiment.
FIG. 8 is an example of a relationship between an air pressure measured by an air pressure sensor and an output voltage.
FIG. 9 is a flowchart illustrating an operation based on a first detection unit of the ultraviolet-light-radiating device according to the first embodiment.
FIG. 10 is a view illustrating a variation example of air pressure, and ON/OFF state of driving signal and the ultraviolet-light-radiating device.
FIG. 11 is a circuit diagram executing the ultraviolet-light-radiating device according to the first embodiment.
FIG. 12 is a view illustrating one example of a configuration of an ultraviolet-light-radiating device according to a second embodiment.
FIG. 13 is a flowchart illustrating operations that the ultraviolet-light-radiating device according to the second embodiment performs based on a first detection unit and a second detection unit.
FIG. 14 is a view illustrating a variation example of air pressure, detection of human presence, and ON/OFF state of driving signal and the ultraviolet-light-radiating device.
FIG. 15 is a circuit diagram executing the ultraviolet-light-radiating device according to the second embodiment.

### [Mode for Carrying out the Invention]

Now, embodiments of the present invention will be described with reference to the drawings. These embodiments are not intended to limit the scope of the present invention. Further, in the drawings, the same portions are denoted with the same reference numbers.

If there are a plurality of components having the same or similar functions, different subscripts may be attached after the same reference number for description. If there is no need to distinguish the plurality of components, the subscripts may be omitted.

The positions, sizes, shapes, and ranges of the respective components illustrated in the drawings are for facilitating the understanding of the present invention, and may not represent the actual positions, sizes, shapes, and ranges of the components. Therefore, the present invention is not limited to the positions, sizes, shapes, and ranges illustrated in the drawings.

At first, a conventional ultraviolet-light-radiating device will be described with reference to FIGs .1 to 6.

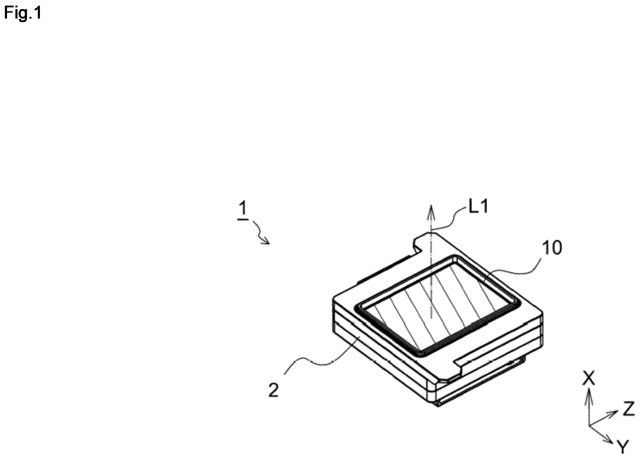

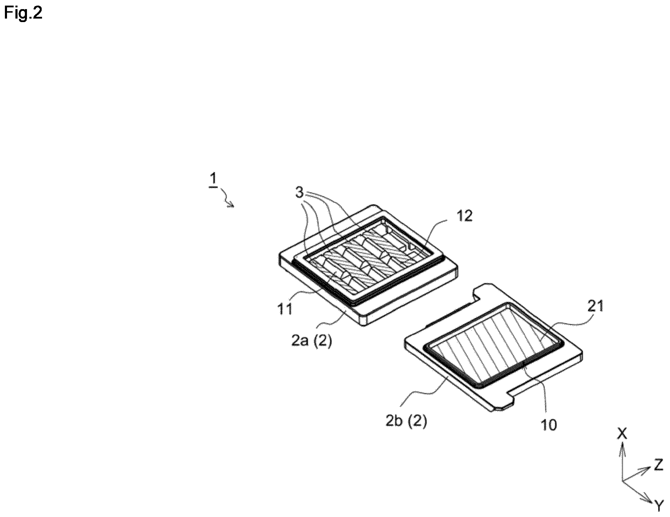

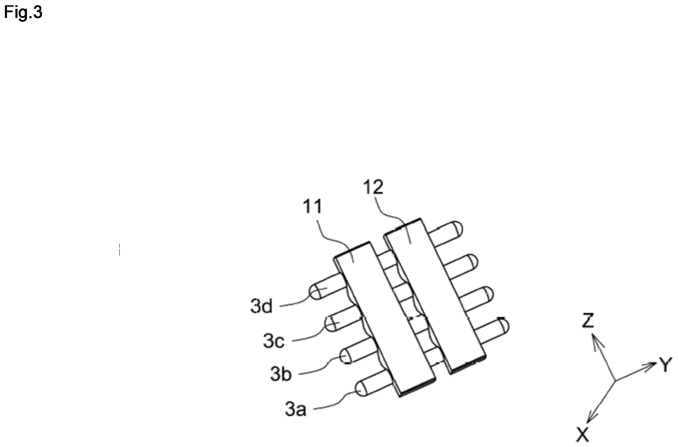

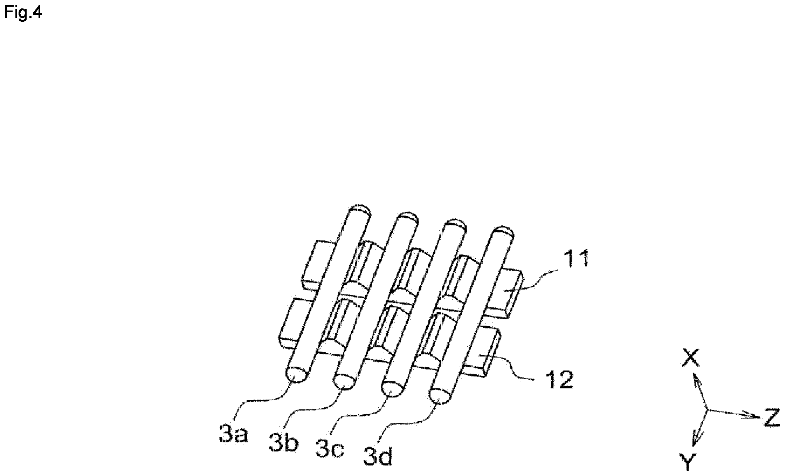

FIG. 1 is a perspective view schematically illustrating an external appearance of an ultraviolet-light-radiating device. Further, FIG. 2 is a perspective view having exploded a main body casing portion 2a and a lid portion 2b of a lamp house 2 from the ultraviolet-light-radiating device of FIG. 1. FIG. 3 is a perspective view schematically illustrating a configuration of an electrode block and excimer lamps included in the ultraviolet-light-radiating device. FIG. 4 is a perspective view having the viewpoint changed from FIG. 3.

In the following drawings, an X-Y-Z coordinate system is referred for explanation, wherein an extraction direction of ultraviolet light L1 is referred to as the X direction, and a plane surface orthogonal to the X direction is the YZ plane surface. In further detail, as will be described in detail below with reference to the drawings of FIG. 4 and subsequent drawings, a tube axis direction of an excimer lamp 3 is set as the Y direction, and directions orthogonal to the X direction and the Y direction is set as the Z direction. The X direction corresponds to a "first direction", the Y direction corresponds to a "second direction", and the Z direction corresponds to a "third direction".

As illustrated in FIGs. 2 and 3, the ultraviolet-light-radiating device is equipped with the lamp house 2 on one surface of which is formed a light extraction surface 10. The lamp house 2 is equipped with the main body casing portion 2a and the lid portion 2b, and within the main body casing portion 2a are accommodated the excimer lamp 3 and electrode blocks (11, 12). In the present embodiment, a case where four excimer lamps 3 (3a, 3b, 3c, 3d) are accommodated in the lamp house 2 is illustrated as an example (refer to FIG. 4), but the number of the excimer lamp 3 may be one, or two, three, or even more than five. The electrode blocks (11, 12) constitute electrodes for feeding power to the respective excimer lamps 3.

In the present embodiment, as illustrated in FIG. 2, an optical filter 21 is provided at an area that constitutes the light extraction surface 10 of the lid portion 2b.

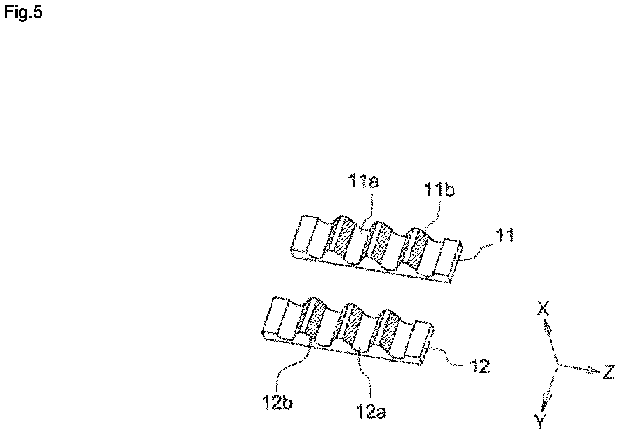

FIGs. 3 and 4 are perspective views that have omitted the main body casing portion 2a from the drawing of FIG. 2 and that only illustrate the electrode blocks (11, 12) and the excimer lamps 3 (3a, 3b, 3c, 3d). FIGs. 3 and 4 only differ in the angles from which the drawings are illustrated. Further, FIG. 5 is a perspective view having omitted the excimer lamp 3 from the drawing of FIG. 4.

As illustrated in FIGs. 3 and 4, an ultraviolet-light-radiating device 1000 according to the present embodiment is equipped with four excimer lamps 3 (3a, 3b, 3c, 3d) that are disposed in a separated manner in the Z direction. Further, two electrode blocks (11, 12) are disposed so as to be in contact with the outer surface of luminous tubes of the respective excimer lamps 3. In the following description, as required, the electrode block 11 is referred to as a "first electrode block 11" and the electrode block 12 is referred to as a "second electrode block 12". These electrode blocks may all be formed of a conductor, or of a conductor and a nonconductive body that are assembled in the form of a block.

The first electrode block 11 and the second electrode block 12 are disposed at positions separated from each other in the Y direction. As illustrated in FIG. 5, the first electrode block 11 may have mounting regions 11a that present a shape along a curved surface that the outer surface of the luminous tubes of the excimer lamps 3 presents and on which the excimer lamps 3 are mounted, and tapered surfaces 11b that are formed at a position separated in the Z direction from the excimer lamps 3 and that are inclined with respect to the YZ plane surface. Similarly, the second electrode block 12 may have mounting regions 12a and tapered surfaces 12b. Thereby, light that has been irradiated from the excimer lamps 3 may easily be radiated in the X direction (however, the present device may function without the tapered surfaces).

The first electrode block 11 and the second electrode block 12 are each composed of a material having conductivity, and preferably, composed of a material that exhibits reflectivity to ultraviolet light L1 of a first wavelength band. As an example, the first electrode block 11 and the second electrode block 12 are both composed of an aluminum, an aluminum alloy, or a stainless steel. Further, a structure may be adopted where a conductor formed of an aluminum, an aluminum alloy, or a stainless steel is arranged on a base made of ceramics.

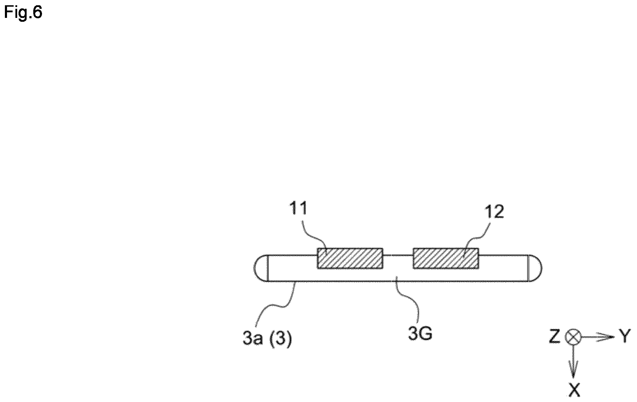

FIG. 6 is a view schematically illustrating a positional relationship of the excimer lamp 3 and the electrode blocks (11, 12), corresponding to a schematical plan view in a state where the excimer lamp 3 is viewed in the +Z direction. In FIG. 6, only the excimer lamp 3a positioned on the most -Z side of the four excimer lamps 3 (3a, 3b, 3c, 3d) is illustrated, and the other excimer lamps (3b, 3c, 3d) are not shown, but as described above, the excimer lamps (3b, 3c, 3d) are also arranged side by side in the +Z direction.

The excimer lamp 3 has a luminous tube in which the Y direction is the tube axis direction, and the outer surface of the luminous tube of the excimer lamp 3 is in contact with each of the electrode blocks (11, 12) at positions separated from each other in the Y direction. A light emitting gas 3G is sealed in the luminous tube of the excimer lamp 3. If a high frequency AC voltage of approximately 10 kHz to 5 MHz is applied between the respective electrode blocks (11, 12), the voltage is applied to the light emitting gas 3G through the luminous tube of the excimer lamp 3. In this state, discharge plasma is generated within a discharge space in which the light emitting gas 3G is sealed, by which atoms in the light emitting gas 3G are excited to realize an excimer state, and excimer emission occurs when these atoms are transferred to a ground state.

The light emitting gas 3G is composed of a material that emits ultraviolet light L1 that belong to a first wavelength band in which the main emission wavelength is 190 nm or more and 225 nm or less when excimer emission occurs. As an example, KrCl, KrBr, and ArF are contained in the light emitting gas 3G. In addition to the above-mentioned gas types, inert gas such as argon (Ar) and neon (Ne) may be mixed therein.

For example, if KrCl is contained in the light emitting gas 3G, ultraviolet light L1 whose main peak wavelength is around 222 nm is emitted from the excimer lamp 3. If KrBr is contained in the light emitting gas 3G, ultraviolet light L1 whose main peak wavelength is around 207 nm is emitted from the excimer lamp 3. If ArF is contained in the light emitting gas 3G, ultraviolet light L1 whose main peak wavelength is around 193 nm is emitted from the excimer lamp 3. The spectrum of ultraviolet light L1 emitted from the excimer lamp 3 that contains KrCl in the light emitting gas 3G is as described above.

If KrCl is contained in the light emitting gas 3G, optical output mainly concentrates near 222 nm, which is the main peak wavelength of the spectrum of ultraviolet light L1, but a very small optical output in the wavelength band of 240 nm or more, which is a matter of concern regarding the effect on the human body, is also observed. Therefore, the optical filter 21 is provided to the region constituting the light extraction surface 10 with the aim to block the light component of the relevant wavelength band.

By adopting such a configuration, the luminous tube may maintain insulation between electrodes even if a high voltage of a kV level is applied when being turned on in a normal atmosphere environment of 1 atm.

As described above, in a conventional ultraviolet-light-radiating device, insulation between electrodes is realized by air insulation. However, insulation strength by air is proportional to its air pressure, such that in a 1-atm environment, a high insulation property is realized, whereas if the air pressure drops, the insulation strength also drops therewith.

Specifically, insulation breakdown occurs if the air pressure falls to a certain air pressure or below, whereas in an ultraviolet-light-radiating device, a voltage of a high kV level is applied between the electrodes, such that in a state below the certain air pressure, discharge that should occur within the excimer lamps may be directly discharged between electrodes and not via the excimer lamps, which may cause excessive electricity to flow within the electric circuit and may lead to malfunction.

### [First Embodiment]

Therefore, according to the first embodiment of the present disclosure, a configuration described below is provided to enable ultraviolet light to be irradiated stably even in a low air pressure environment.

In the following description, components that are the same or equivalent as those of the conventional ultraviolet-light-radiating device described above are denoted with the same reference numbers, and descriptions thereof are either simplified or omitted.

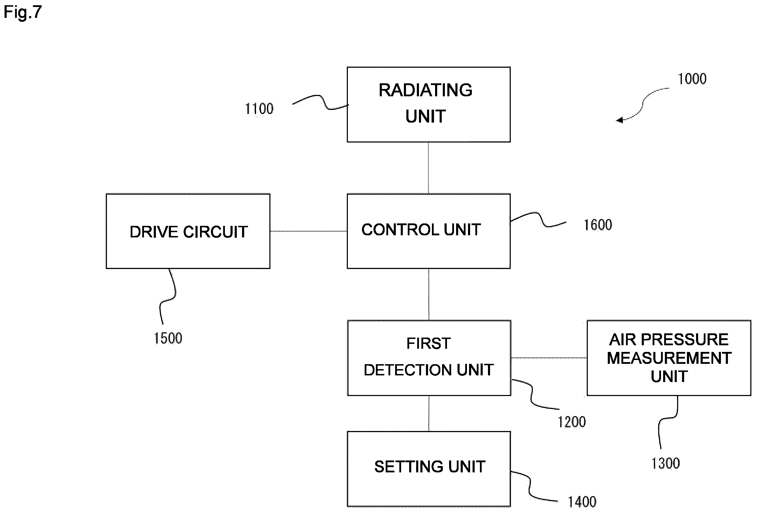

At first, a first embodiment will be described with reference to FIG. 7.

FIG. 7 is a view illustrating one example of a configuration of the ultraviolet-light-radiating device 1000 according to the embodiment of the present disclosure.

The ultraviolet-light-radiating device 1000 is a device capable of irradiating ultraviolet light stably even in a low air pressure environment.

The ultraviolet-light-radiating device 1000 mainly includes a radiating unit 1100, a first detection unit 1200, an air pressure measurement unit 1300 connected to the first detection unit 1200, a setting unit 1400, a drive circuit 1500, and a control unit 1600.

The radiating unit 1100 is connected to the control unit 1600, and irradiates ultraviolet light based on the control of the control unit 1600.

The first detection unit 1200 is connected to the setting unit 1400, and based on a threshold value set by the setting unit 1400, it determines the air pressure of ambient atmosphere.

The radiating unit 1100 is a light source that emits ultraviolet light of a specific wavelength range that belongs within a wavelength range of 190 nm to 225 nm. A rated lamp power of the radiating unit 1100 is a few ten W, such as 20 W or less.

A first electrode 1112 and a second electrode 1113 are formed of a material having conductivity, such as a material that exhibits reflectivity to ultraviolet light of a specific wavelength range that belongs to a wavelength range of 190 nm to 225 nm, for example.

The material may be aluminum, aluminum alloy, or stainless-steel alloy, for example.

The first electrode 1112 and the second electrode 1113 are formed of a conductive material, but they may be formed of other materials. For example, they may be formed of a conductor and a nonconductive body by disposing aluminum, aluminum alloy, or a stainless-steel alloy on a base formed of ceramics.

### <First Detection Unit>

Next, a configuration of the first detection unit 1200 illustrated in FIG. 7 will be described.

The first detection unit 1200 is a function unit that detects a dielectric strength of air in the atmosphere in which the ultraviolet-light-radiating device 1000 is disposed. Specifically, the first detection unit 1200 is connected to the air pressure measurement unit 1300, and detects the dielectric strength of air in the atmosphere in which the ultraviolet-light-radiating device 1000 is disposed based on the air pressure measured by the air pressure measurement unit 1300.

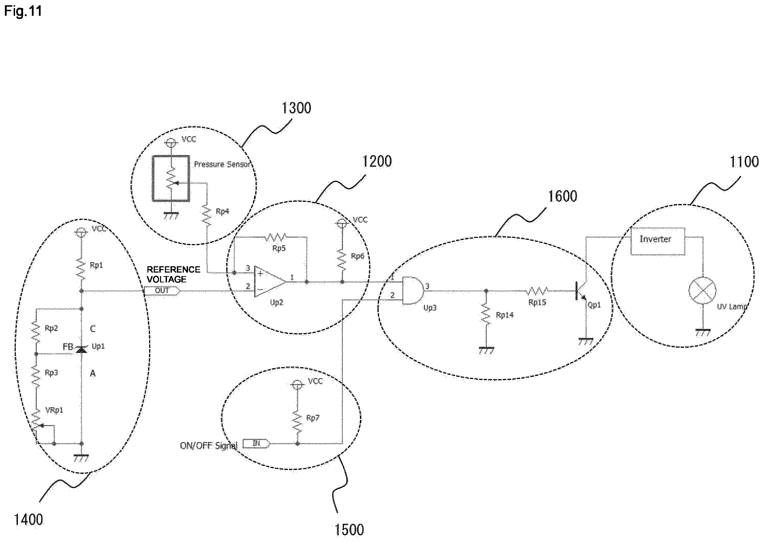

By adopting a circuit configuration as illustrated in FIG. 11 described later, for example, the first detection unit 1200 determines a magnitude of ambient atmospheric pressure and the threshold value by performing comparison with a reference voltage that may be adjusted to a voltage corresponding to 0.0 to 0.8 atm in volume resistance.

The first detection unit may adopt other methods, as long as it enables to compare the magnitude of ambient atmospheric pressure and the threshold value. For example, the method may involve changing the value of a fixed resistance.

The first detection unit 1200 detects whether the dielectric strength of air in the atmosphere in which the ultraviolet-light-radiating device 1000 is disposed is equal to or below a threshold value set arbitrarily.

The first detection unit 1200 computes the air pressure of the atmosphere in which the ultraviolet-light-radiating device 1000 is disposed based on a voltage value transmitted from the air pressure measurement unit 1300.

The first detection unit 1200 is connected to the control unit 1600 and transmits the detected result thereto.

### <Air Pressure Measurement Unit>

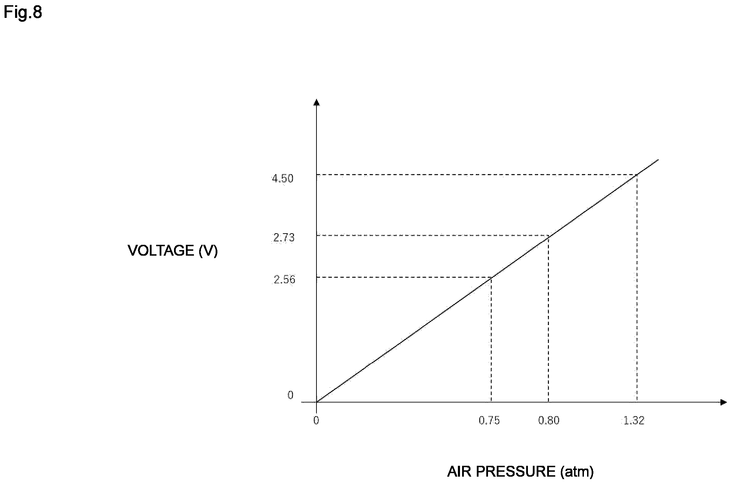

Next, the air pressure measurement unit will be described with reference to FIG. 8. A common air pressure sensor may be used as the air pressure measurement unit. FIG. 8 is a drawing illustrating a relationship between the air pressure being measured by the air pressure sensor and the output voltage.

The air pressure measurement unit 1300 outputs the measurement result by a voltage value of 0 to 5 V, for example, based on the measured air pressure. The air pressure measurement unit 1300 outputs a voltage value of 0 V at 0 atm and 4.5 V at 1.32 atm, for example.

According to the air pressure measurement unit 1300, the output (voltage value) of the air pressure sensor between 0 atm and 1.32 atm may be illustrated as a linear function graph that varies linearly, as illustrated in FIG. 8.

The air pressure measurement unit 1300 transmits the information on the voltage value being output to the first detection unit 1200.

The configuration of the air pressure sensor is well known, such that detailed descriptions of the configuration will be omitted.

### <Setting Unit>

The setting unit 1400 sets the threshold value arbitrarily so as to set up an optimum value for purposes other than in an aircraft cabin.

The setting unit 1400 transmits the threshold value being set arbitrarily to the first detection unit 1200.

The setting unit 1400 is, for example, a rotary switch. The setting unit 1400 may set the threshold value arbitrarily by setting up an arbitrary value for the rotary switch.

The setting unit 1400 is, for example, a numeric key through which an arbitrary value may be entered. By having an arbitrary value entered therethrough, the setting unit 1400 may set the entered value as the threshold value.

The threshold value entered to the setting unit 1400 may be of any unit, as long as the first detection unit 1200 may determine the threshold value.

For example, according to the present embodiment, 0.75 atm is set as the threshold value, but the unit may be the dielectric strength of air of gas.

### <Drive Circuit>

Next, the drive circuit 1500 will be described. The drive circuit 1500 generates a driving signal for varying the irradiation of ultraviolet light at regular intervals to prevent temperature rise of the radiating unit 1100 accompanying the irradiation of ultraviolet light. The drive circuit 1500 is connected to the control unit 1600.

Specifically, the drive circuit 1500 generates an ON signal and an OFF signal that indicate the states of ON and OFF of ultraviolet light irradiated by the ultraviolet-light-radiating device 1000.

According to the present embodiment, the drive circuit 1500 repeatedly generates ON signals and OFF signals at one-minute intervals, but the interval may be other time intervals. For example, if rising of temperature of the radiating unit 1100 may be prevented by a shorter stop time, the ON signals and OFF signals may be repeatedly generated at shorter intervals. For example, if the radiating unit 1100 requires a longer stop time to prevent the rising of temperature, the ON signals and OFF signals may be repeatedly generated with longer intervals.

According to the present embodiment, the rising of temperature of the radiating unit 1100 may be prevented by the drive circuit 1500, but the rising of temperature may also be prevented by other methods.

For example, a cooling circuit for cooling the radiating unit 1100 may be adopted to prevent temperature rise.

### <Control Unit>

Next, the control unit 1600 will be described. The control unit 1600 is a function unit that controls the radiating unit 1100, and it is connected to the radiating unit 1100, the first detection unit 1200, and the drive circuit 1500. The control unit 1600 receives a detection result from the first detection unit 1200. The control unit 1600 receives a driving signal from the drive circuit 1500.

Further, if the dielectric strength of air detected by the first detection unit 1200 is less than the threshold value, for example, the control unit 1600 performs control such that the radiating unit 1100 stops irradiation of ultraviolet light.

Further, if the dielectric strength of air detected by the first detection unit 1200 is equal to or greater than the threshold value, for example, the control unit 1600 performs control such that the radiating unit 1100 irradiates ultraviolet light.

Further, for example, the control unit 1600 performs control such that the radiating unit 1100 irradiates ultraviolet light based on an ON signal generated by the drive circuit 1500.

Furthermore, for example, the control unit 1600 performs control such that the radiating unit 1100 stops irradiation of ultraviolet light based on an OFF signal generated by the drive circuit 1500.

Moreover, if the content of instruction of the irradiation of ultraviolet light based on the first detection unit 1200 and the content of instruction of the irradiation of ultraviolet light based on the drive circuit 1500 differ, the control unit 1600 irradiates ultraviolet light only when contents of instruction of both the first detection unit 1200 and the drive circuit 1500 instruct irradiation of ultraviolet light.

For example, when the dielectric strength of air detected by the first detection unit 1200 is smaller than the threshold value and when the drive circuit 1500 generates an ON signal, the irradiation of ultraviolet light is stopped.

### <Operation of Ultraviolet-Light-Radiating Device>

Next, an operation of the ultraviolet-light-radiating device 1000 according to the present embodiment will be described.

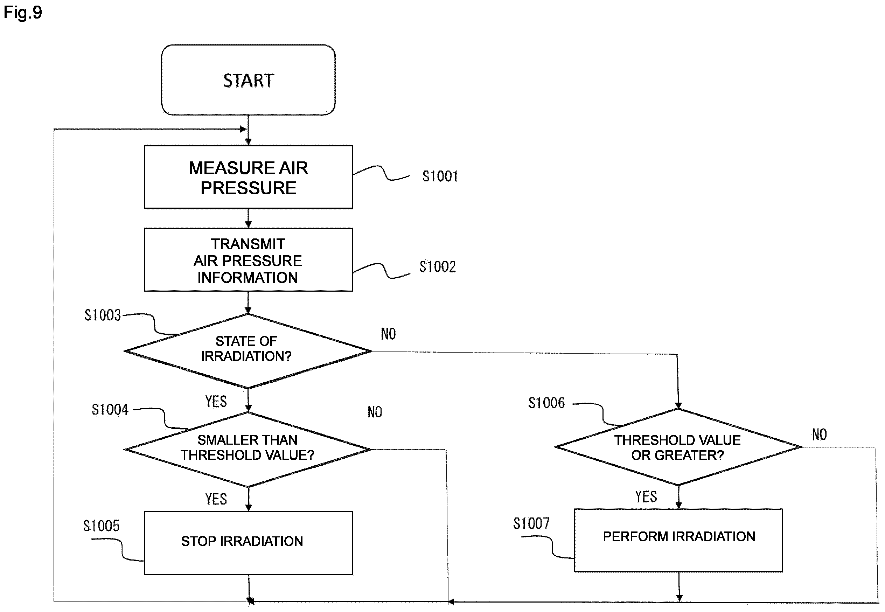

FIG. 9 is a flowchart illustrating an operation that the ultraviolet-light-radiating device 1000 according to the present embodiment performs based on the first detection unit 1200.

Hereafter, the processes executed by the ultraviolet-light-radiating device 1000 will be described with reference to the flowchart of FIG. 9.

At first, the air pressure measurement unit 1300 measures the air pressure of the atmosphere in which the ultraviolet-light-radiating device 1000 is disposed (S1001).

Specifically, the air pressure measurement unit 1300 outputs the voltage value corresponding to the air pressure of the atmosphere in which the ultraviolet-light-radiating device 1000 is disposed.

Next, the air pressure measurement unit 1300 transmits the information of the air pressure being measured to the first detection unit 1200 (S1002).

Specifically, the air pressure measurement unit 1300 transmits the information of the voltage value being output to the first detection unit 1200.

Next, the control unit 1600 determines the state of irradiation of ultraviolet light (S1003).

Specifically, the control unit 1600 determines whether the radiating unit 1100 is irradiating ultraviolet light. If ultraviolet light is irradiated, the procedure advances to step S1004, and if not, the procedure advances to step S1006.

Next, the first detection unit 1200 determines whether the air pressure is smaller than a threshold value (S1004).

Specifically, the first detection unit 1200 computes the air pressure corresponding to the voltage value received from the air pressure measurement unit 1300. The first detection unit 1200 determines whether the computed pressure is smaller than the set threshold value. The first detection unit 1200 transmits the determined result to the control unit 1600.

If the value is smaller than the set threshold value, the procedure advances to step S1005, and if the value is equal to or greater than the set threshold value, the procedure returns to step S1001 and repeats the processing.

Next, the control unit 1600 stops irradiating ultraviolet light (S1005).

Specifically, the control unit 1600 stops irradiation of ultraviolet light based on the determined result received from the first detection unit 1200, returns to step S1001, and repeats the processing.

Next, the first detection unit 1200 determines whether the air pressure is equal to or greater than the threshold value (S1006).

Specifically, the first detection unit 1200 computes the air pressure corresponding to the voltage value received from the air pressure measurement unit 1300. The first detection unit 1200 determines whether the computed pressure is equal to or greater than the set threshold value. The first detection unit 1200 transmits the determined result to the control unit 1600.

If the value is equal to or greater than the set threshold value, the procedure advances to step S1007, and if the value is smaller than the set threshold value, the procedure returns to step S1001 and repeats the processing.

Next, the control unit 1600 irradiates ultraviolet light (S1007).

Specifically, the control unit 1600 irradiates ultraviolet light based on the determined result received from the first detection unit 1200, returns to step S1001, and repeats the processing.

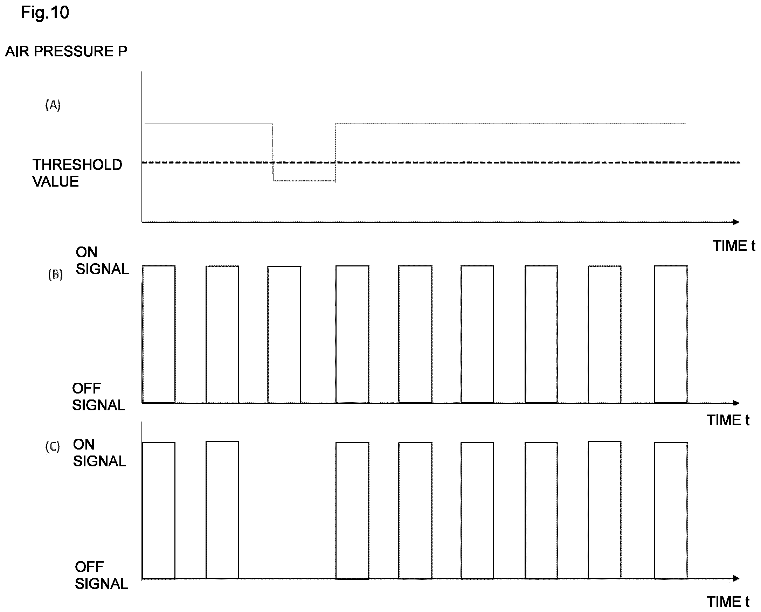

Next, an example of a specific relationship between air pressure and irradiation of ultraviolet light in a case where the steps described above have been adopted will be described with reference to FIG. 10.

A graph (A) illustrated on the upper level of FIG. 10 indicates a variation example of the air pressure. A graph (B) illustrated on the middle level thereof indicates a state of driving signals generated by the drive circuit 1500. A graph (C) on the lower level thereof indicates an ON/OFF state of the ultraviolet-light-radiating device 1000. According to the graph (C) on the lower level, when the driving signal of the drive circuit 1500 is an ON signal, the ultraviolet-light-radiating device is in an ON state, and when the driving signal is an OFF signal, the ultraviolet-light-radiating device is in an OFF state. In all the graphs, the horizontal axis indicates time.

As can be recognized from this graph, in principle, the ultraviolet-light-radiating device 1000 repeatedly irradiates and stops irradiating ultraviolet light based on a driving signal generated by the drive circuit 1500.

In a state where the driving signal is ON and the air pressure is equal to or greater than the threshold value, the ultraviolet-light-radiating device 1000 is in an ON state, but regardless of the state of the driving signal, once the air pressure falls below the threshold value, the ultraviolet-light-radiating device 1000 is turned OFF. In a state where the air pressure is once again returned to the threshold value or greater and the driving signal generates an ON signal, the ultraviolet-light-radiating device 1000 is varied and returned to the ON state. For example, a case where the ultraviolet-light-radiating device 1000 is installed on an aircraft will be described.

The aircraft on which the ultraviolet-light-radiating device 1000 is installed stands by on the ground before takeoff. The aircraft flies in the air after takeoff, and lands when its destination has been reached. The aircraft repeats this sequence of operations.

When the aircraft is on the ground, the pressure inside the cabin and the pressure outside the aircraft are both approximately 1 atm, but when the aircraft is flying in the air, such as at an altitude of 10,000 m, the pressure is approximately 0.8 atm inside the cabin whereas the pressure drops to 0.2 atm outside the aircraft.

If the set threshold value is 0.75 atm, the ultraviolet-light-radiating device 1000 repeatedly irradiates and stops irradiating ultraviolet light based on the driving signal generated by the drive circuit 1500.

In addition to a case where the drive circuit 1500 generates an OFF signal as the driving signal, the ultraviolet-light-radiating device 1000 stops irradiating ultraviolet light, for example, in a case where the device that controls the air pressure within the aircraft malfunctions and the air pressure within the cabin temporarily drops to an air pressure below 0.8 atm, or in a case where an accident occurs where the body of the aircraft is pierced and air is leaked therethrough.

In such a case, as illustrated in FIG. 10, an event occurs in which the air pressure drops below the threshold value. In such a case, there is a risk that an insulation breakdown may occur between electrodes of the ultraviolet-light-radiating device, such that the irradiation of ultraviolet light will stop.

Thereafter, when the control of air pressure of the cabin is recovered, that is, when the air pressure has returned to the threshold value or more, irradiation of ultraviolet light is restarted.

As described, according to the ultraviolet-light-radiating device 1000 of the present embodiment, irradiation of ultraviolet light may be stopped automatically and restarted automatically according to the state of ambient air pressure of the ultraviolet-light-radiating device 1000.

Therefore, if the dielectric strength of air has dropped along with the dropping of air pressure, it may be possible to prevent abnormal discharge from occurring. That is, even in a location where the air pressure varies based on a regular form of use, the ultraviolet-light-radiating device 1000 having a compact size may be utilized stably.

As precautionary measures against abnormal discharge, the following measures may be taken, but such measures may have various problems as described below, such that the method illustrated in the present embodiment is advantageous.

### (Extension of Distance between Electrodes)

As precautionary measures against abnormal discharge, for example, the electrodes may be arranged such that distance between the electrodes is extended. Thereby, the amount of air existing between the electrodes may be increased and the insulation strength may be enhanced. However, insulation distance of the excimer lamps 3 will also be extended by taking such measures, according to which the starting of discharge of the excimer lamps 3 becomes difficult, and in the worst case, the excimer lamps 3 will not be turned on. Further, by adopting such measures, a certain distance between the electrodes is required, such that the size of the radiating unit 1100 may be increased, and as a result, the size of the ultraviolet-light-radiating device 1000 is increased.

### (Loading of Insulating Material)

As precautionary measures against abnormal discharge, for example, an insulating material having a higher insulation strength than air is loaded between electrodes. The insulating material may be, for example, Teflon, plastic, and vinyl insulating materials that exhibit electrical insulation characteristics. Thereby, the insulation strength may be increased. According to such measures, the insulating material will be disposed for all the electrodes, such that the reduction of weight will be deteriorated.

### (Introduction of Protection Circuit)

A protection circuit such as a fuse is adopted as precautionary measures against abnormal discharge. Thereby, when abnormal discharge occurs, the fuse is activated and a circuit is opened, such that abnormal discharge may be prevented from flowing to the entirety of the ultraviolet-light-radiating device 1000.

According to such measures, when the fuse is activated once, the fuse must be exchanged with a conforming product to be used again. Even in a case of a restorable fuse, an operation to restore the fuse by an operator is required.

The mechanism of the fuse requires overcurrent to be supplied for a certain amount of time before it is activated, during which time an overcurrent that exceeds the current assumed in the design stage is applied to electronic components around the fuse. Thereby, stress is applied to the electronic components other than the fuse, which may cause other malfunctions.

### (Emergency Monitoring Device)

Various sensors may be installed as precautionary measures against abnormal discharge. Thereby, the radiating unit 1100 may be stopped when abnormal discharge occurs.

According to such measures, a monitoring operator for monitoring outputs of various sensors, or a monitoring software that replaces the monitoring operator, and a communication network for transmitting the sensor output to a server, are required.

### (Examples)

Next, an example of a circuit configuration for realizing the embodiment of the present disclosure will be described with reference to FIG. 11.

FIG. 11 is a circuit diagram that implements the ultraviolet-light-radiating device 1000.

The components included in the ultraviolet-light-radiating device 1000 are implemented by basic electronic components as illustrated in FIG. 11. Basic electronic components may include an AND gate, a resistor, a diode, and a comparator.

The ultraviolet-light-radiating device 1000 is composed by combining basic electronic components.

For example, the first detection unit 1200 compares the voltage value output from the air pressure measurement unit 1300 and the reference voltage output from the setting unit 1400 using a comparator. If the voltage value is greater than the reference voltage, a logical value 1 is output, and if smaller, a logical value 0 is output.

The reference voltage is determined by changing the value of the volume resistance composed by the setting unit 1400.

For example, the drive circuit 1500 outputs a logical value 1 when a ON signal is generated, and outputs a logical value 0 when an OFF signal is generated.

The control unit 1600 outputs a logical value 1 from the AND gate when both the logical value output from the first detection unit 1200 and the logical value output from the drive circuit 1500 are 1.

The control unit 1600 outputs a logical value 1 such that ultraviolet light is irradiated on the radiating unit 1100.

### [Second Embodiment]

An ultraviolet-light-radiating device 2000 according to the second embodiment differs from the first embodiment in that the device is equipped with a function to stop irradiation of ultraviolet light when a human presence is detected, in addition to air pressure.

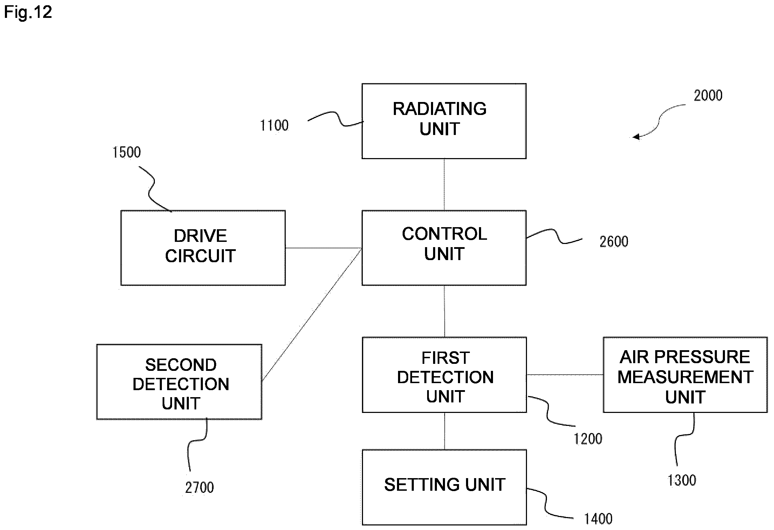

FIG. 12 is a view illustrating one example of a configuration of the ultraviolet-light-radiating device 2000 according to an embodiment of the present disclosure.

In the following description, configurations that are the same or equivalent to those of the first embodiment are denoted with the same reference numbers, and descriptions thereof are omitted or simplified.

The ultraviolet-light-radiating device 2000 mainly includes the radiating unit 1100, the first detection unit 1200, the air pressure measurement unit 1300 connected to the first detection unit 1200, the setting unit 1400, the drive circuit 1500, a control unit 2600, and a second detection unit 2700.

### <Second Detection Unit>

Next, a configuration of the second detection unit 2700 illustrated in FIG. 12 will be described.

The second detection unit 2700 is a function unit that detects presence of a human at a location where the ultraviolet-light-radiating device 2000 is disposed. Specifically, the second detection unit 2700 is connected to the control unit 2600, and detects a human presence in the region detected by the second detection unit 2700.

The second detection unit 2700 detects a human presence in a specific detection range. A specific detection range detected by the second detection unit 2700 refers, for example, to a range in which ultraviolet light is irradiated by the ultraviolet-light-radiating device 2000.

The second detection unit 2700 is, for example, a human motion detector.

When a human presence within the detection range is detected, the second detection unit 2700 transmits information that a human presence has been detected to the control unit 2600.

### <Control Unit>

Next, the control unit 2600 will be described. The control unit 2600 is a function unit that controls the radiating unit 1100, and it is connected to the radiating unit 1100, and to the first detection unit 1200, the drive circuit 1500, and the second detection unit 2700. The control unit 2600 receives detection results from the first detection unit 1200. The control unit 2600 receives driving signals from the drive circuit 1500. The control unit 2600 receives detection results from the second detection unit 2700.

Further, if the dielectric strength of air detected by the first detection unit 1200 is smaller than the threshold value, for example, the control unit 2600 performs control such that the radiating unit 1100 stops irradiation of ultraviolet light.

Furthermore, if the dielectric strength of air detected by the first detection unit 1200 is greater than the threshold value, for example, the control unit 2600 performs control such that the radiating unit 1100 irradiates ultraviolet light.

Moreover, the control unit 2600 performs control such that the radiating unit 1100 irradiates ultraviolet light based on an ON signal generated by the drive circuit 1500.

Further, the control unit 2600 performs control such that the radiating unit 1100 stops irradiation of ultraviolet light based on an OFF signal generated by the drive circuit 1500.

Further, the control unit 2600 performs control such that the radiating unit 1100 stops irradiation of ultraviolet light when the second detection unit 2700 detects human presence.

Moreover, the control unit 2600 performs control such that the radiating unit 1100 irradiates ultraviolet light when the second detection unit 2700 does not detect human presence.

Furthermore, if the content of instruction of irradiation of ultraviolet light based on the first detection unit 1200, the content of instruction of irradiation of ultraviolet light based on the drive circuit 1500, and the content of instruction of irradiation of ultraviolet light based on the second detection unit 2700 differ, the control unit 2600 irradiates ultraviolet light only when all the contents of instruction of the first detection unit 1200, the drive circuit 1500, and the second detection unit 2700 instruct irradiation of ultraviolet light.

For example, if the dielectric strength of air detected by the first detection unit 1200 is smaller than the threshold value and if the second detection unit 2700 detects human presence, the irradiation of ultraviolet light is stopped.

Next, the operation of the ultraviolet-light-radiating device 2000 according to the present embodiment will be described.

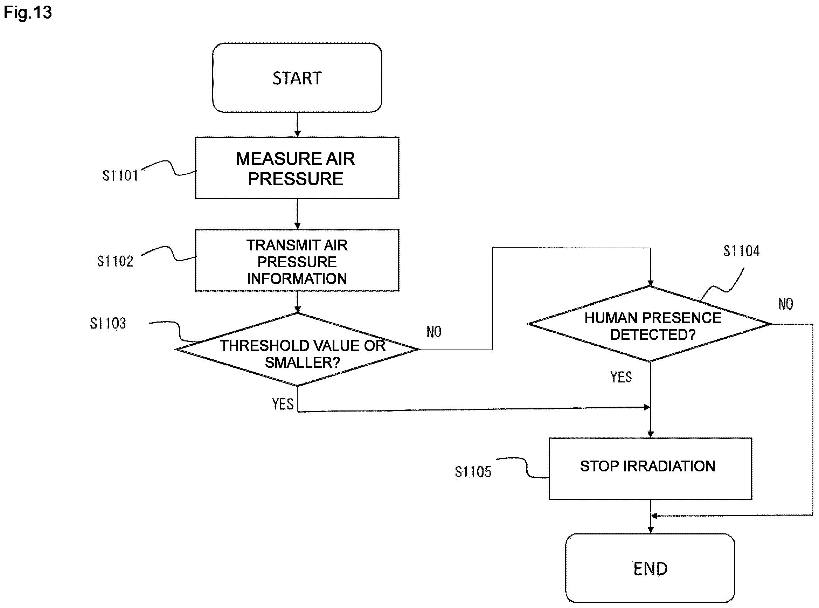

FIG. 13 is a flowchart illustrating an operation of the ultraviolet-light-radiating device 2000 according to the present embodiment based on the first detection unit 1200 and the second detection unit 2700.

Hereafter, the processing executed by the ultraviolet-light-radiating device 2000 will be described with reference to the flowchart of FIG. 13. The flowchart of FIG. 13 is started when the ultraviolet-light-radiating device 2000 irradiates ultraviolet light in an environment of 0.75 atm or higher.

At first, the air pressure measurement unit 1300 measures an air pressure of the atmosphere in which the ultraviolet-light-radiating device 2000 is disposed (S1101).

Specifically, the air pressure measurement unit 1300 outputs a voltage value corresponding to the air pressure of the atmosphere in which the ultraviolet-light-radiating device 2000 is disposed.

Next, the air pressure measurement unit 1300 transmits the information of the air pressure being measured to the first detection unit 1200 (S1102).

Specifically, the air pressure measurement unit 1300 transmits the information of the voltage value being output to the first detection unit 1200.

Next, the first detection unit 1200 determines whether the air pressure is smaller than the threshold value (S1103).

Specifically, the first detection unit 1200 computes the air pressure corresponding to the voltage value received from the air pressure measurement unit 1300. The first detection unit 1200 determines whether the computed pressure is smaller than the threshold value being set. The first detection unit 1200 transmits the determined result to the control unit 2600.

If the value is smaller than the threshold value being set, the procedure advances to step S1105, whereas if the value is equal to or greater than the threshold value being set, the procedure advances to step S1104.

Next, the second detection unit 2700 determines whether human presence has been detected (S1104).

Specifically, the second detection unit 2700 determines whether human presence within a specific detection range has been detected. When human presence has been detected, the second detection unit 2700 transmits the information to the control unit 2600.

Next, the control unit 2600 stops the irradiation of ultraviolet light (S1105).

Specifically, the control unit 2600 stops the irradiation of ultraviolet light based on the determined result received from the first detection unit 1200 and the second detection unit 2700.

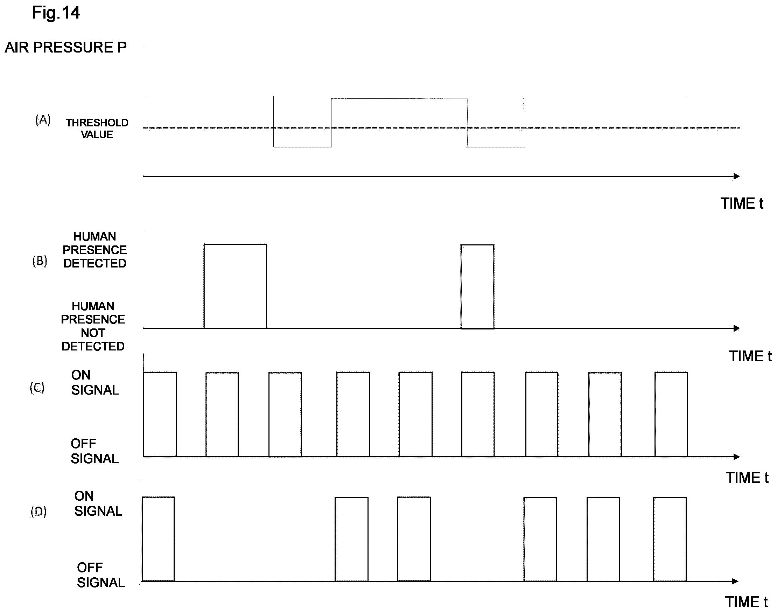

Next, an example of a specific relationship between the air pressure and irradiation of ultraviolet light in a case where the steps described above have been adopted will be described with reference to FIG. 14.

A graph (A) illustrated on the upper level of FIG. 14 indicates a variation example of the air pressure. A graph (B) illustrated on the middle level thereof indicates a variation example of the detection of human presence. A graph (C) illustrated on the middle level thereof indicates a state of the driving signals generated by the drive circuit 1500. A graph (D) illustrated on the lower level thereof indicates an ON/OFF state of the ultraviolet-light-radiating device. In all the graphs, the horizontal axis represents time. According to the graph (D) on the lower level, when the driving signal is an ON signal, the ultraviolet-light-radiating device is in an ON state, and when the driving signal is an OFF signal, the ultraviolet-light-radiating device is in an OFF state.

As can be recognized from this graph, in principle, the ultraviolet-light-radiating device 2000 repeatedly irradiates and stops irradiating ultraviolet light based on a driving signal generated by the drive circuit 1500.

The control unit 2600 controls the radiating unit 1100 based on the information detected by the first detection unit 1200 and the information detected by the second detection unit 2700.

When the control unit 2600 receives either an information that the first detection unit 1200 has detected that the dielectric strength of air in the atmosphere in which the ultraviolet-light-radiating device 2000 is disposed is equal to the threshold value being set arbitrarily or less, or an information that the second detection unit 2700 has detected human presence, the control unit 2600 stops the irradiation of ultraviolet light.

As can be recognized from this graph, if the air pressure is equal to or greater than the threshold value, the ultraviolet-light-radiating device is in an ON state, whereas once the air pressure falls to or below the threshold value, the ultraviolet-light-radiating device will be in an OFF state, and if the air pressure returns again to the threshold value or greater, the ultraviolet-light-radiating device is also returned to the ON state.

In addition to the conditions described above, according to the ultraviolet-light-radiating device 2000 of the present embodiment, even if human presence has been detected, the ultraviolet-light-radiating device will be in an OFF state, and when the air pressure returns to the threshold value or greater and human presence is not detected, the ultraviolet-light-radiating device is set to be returned to the ON state.

According to the ultraviolet-light-radiating device 2000, in a state where the driving signal is an ON signal, the air pressure is equal to or greater than the threshold value, and human presence is not detected, it will be in an ON state, but regardless of the state of the driving signal, once the air pressure falls to or below the threshold value, or when a human presence is detected, the ultraviolet-light-radiating device 2000 will be in an OFF state.

The ultraviolet-light-radiating device 2000 is designed such that when the air pressure is returned to the threshold value or greater, the driving signal generates an ON signal, and further when human presence is not detected, the ultraviolet-light-radiating device is set to be returned to the ON state.

### (Example)

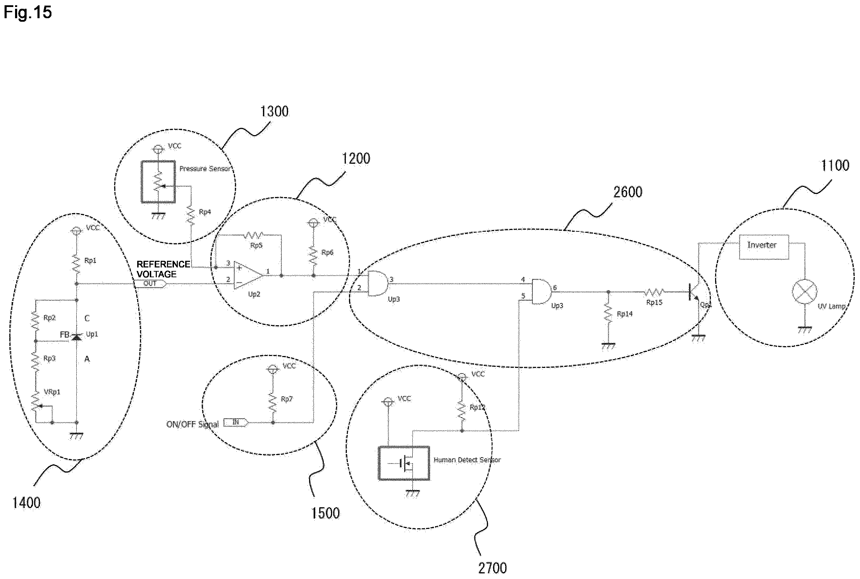

Next, an example of a circuit configuration for realizing an embodiment of a present embodiment according to the present disclosure will be described with reference to FIG. 15.

FIG. 15 is a circuit diagram for implementing the ultraviolet-light-radiating device 2000.

The components included in the ultraviolet-light-radiating device 2000 are implemented by basic electronic components as illustrated in FIG. 15. Basic electronic components may include an AND gate, a resistor, a diode, and a comparator.

The ultraviolet-light-radiating device 2000 is composed by combining basic electronic components.

For example, the first detection unit 1200 compares the voltage value output from the air pressure measurement unit 1300 and the reference voltage output from the setting unit 1400 using a comparator. If the voltage value is greater than the reference voltage, a logical value 1 is output, and if smaller, a logical value 0 is output.

The reference voltage is determined by changing the value of the volume resistance composed by the setting unit 1400.

For example, the drive circuit 1500 outputs a logical value 1 when an ON signal is generated, and the drive circuit 1500 outputs a logical value 0 when an OFF signal is generated.

For example, the second detection unit 2700 outputs a logical value 1 if human presence is not detected, and outputs a logical value 0 if human presence is detected.

The control unit 2600 outputs a logical value 1 from the AND gate when both the logical value output from the first detection unit 1200 and the logical value output from the drive circuit 1500 are 1.

Further, the control unit 2600 outputs a logical value 1 from the AND gate when both the logical value output from the AND gate and the logical value output from the second detection unit 2700 are 1.

The control unit 2600 outputs a logical value 1 such that ultraviolet light is irradiated by the radiating unit 110 when both the logical values output from the AND gate are 1.

As described, according to the ultraviolet-light-radiating device 2000 of the present embodiment, in addition to the state of ambient air pressure of the ultraviolet-light-radiating device 2000, the irradiation of ultraviolet light may be stopped automatically when human presence is detected, and resumed automatically.

Thereby, even if a human is present within the range in which the ultraviolet-light-radiating device 2000 irradiates ultraviolet light, it may be possible to prevent ultraviolet light from being irradiated on the human body. That is, even in a location where human presence occurs in the normal form of use, the ultraviolet-light-radiating device 2000 may be utilized safely.

The embodiments of the present invention have been described above, but the present invention is not limited to the embodiments descried above, and various modifications may be made within the range not deviating from the scope of the present invention.

### [Description of the Reference Numeral]

1000: ultraviolet-light-radiating device, 1100: radiating unit, 1112: first electrode, 1113: second electrode, 1200: first detection unit, 1300: air pressure measurement unit, 1400: setting unit, 1500: drive circuit, 1600, 2600: control unit, 2700: second detection unit

## Claims

1. An ultraviolet-light-radiating device comprising:
a radiating unit configured to irradiate ultraviolet light;
a first detection unit configured to detect a dielectric strength of air in an atmosphere in which the ultraviolet-light-radiating device is disposed; and
a control unit configured to control the radiating unit based on the dielectric strength of air detected by the first detection unit.

2. The ultraviolet-light-radiating device according to claim 1,
wherein the first detection unit transmits a detection result to the control unit in a state where the dielectric strength of air being detected is smaller than a threshold value, and
wherein the control unit stops irradiation of ultraviolet light based on the detection result being received.

3. The ultraviolet-light-radiating device according to claim 2, further comprising
a setting unit configured to set the threshold value of the dielectric strength of air to an arbitrary value.

4. The ultraviolet-light-radiating device according to claim 1, further comprising
an air pressure measurement unit configured to measure an air pressure of the atmosphere in which the ultraviolet-light-radiating device is disposed,
wherein the first detection unit is configured to detect the dielectric strength of air based on the air pressure measured by the air pressure measurement unit.

5. The ultraviolet-light-radiating device according to claim 1, further comprising
a second detection unit configured to detect a human presence.

6. The ultraviolet-light-radiating device according to claim 1,
wherein the radiating unit includes a first electrode disposed in contact with an outer surface of a luminous tube, and a second electrode disposed in contact with an outer surface of the luminous tube at a position separated in a direction parallel to a tube axis from the first electrode, and the radiating unit is configured to irradiate ultraviolet light of a specific wavelength belonging to a wavelength range between 190 nm and 225 nm.

7. The ultraviolet-light-radiating device according to claim 2,
wherein the first detection unit is configured such that the threshold value is set equal to or smaller than 0.8 atm.

8. The ultraviolet-light-radiating device according to claim 2,
wherein the first detection unit is configured such that the threshold value is set to 0.75 atm.

9. A method for controlling ultraviolet-light-radiating device, the method comprising:
regarding the ultraviolet-light-radiating device according to claim 1,
detecting a dielectric strength of air in the atmosphere in which the ultraviolet-light-radiating device is disposed; and
controlling irradiation of ultraviolet light based on the dielectric strength of air being detected.
